# EUROPEAN PATENT APPLICATION

(11) **EP 4 122 392 A1**
(43) Date of publication of application: **25.01.2023**
(21) Application number: 22185503.4
(22) Date of filing: 18.07.2022
(51) Int. Cl.: A61B 5/11, A61B 5/00, G01P 15/00

(54) **AUTOMATED DETECTION OF HEAD AFFECTING IMPACT EVENTS IN DATA COLLECTED VIA INSTRUMENTED MOUTHGUARD DEVICES**

(30) Priority: 19.07.2021 AU 2021902210
(71) Applicant: HitIQ Limited, South Melbourne, Victoria 3205 (AU)
(72) Inventor: ERIKSON, David, South Melbourne, 3205 (AU); VEGAR, Mike, South Melbourne, 3205 (AU); GOODIN, Peter, South Melbourne, 3205 (AU)
(74) Representative: Doherty, William

(57) **Abstract**

The present invention relates, in various embodiments, to automated detection of head and/or head-affecting body impact events in data collected via instrumented mouthguard devices. For example, in some embodiments the invention relates to training and operation of an impact classifier system, which is configured to identify head affecting impacts from time-series data collected by an instrumented mouthguard device. Some embodiments relate to a two-stage method for processing impacts, including a first stage in which a set of data is classified by such an impact classifier system, and a second stage whereby impacts classified as head affecting impacts are designated a numerical value based on a predefined scale.

## Description

### FIELD OF THE INVENTION

The present invention relates, in various embodiments, to automated detection of head affecting impact events (i.e. head impacts and/or body impacts which affect the head) in data collected via instrumented mouthguard devices. For example, in some embodiments the invention relates to training and operation of an impact classifier system, which is configured to identify head impacts from time-series data collected by an instrumented mouthguard device. Some embodiments relate to a two-stage method for processing impacts, including a first stage in which a set of data is classified by such an impact classifier system, and a second stage whereby impacts classified as head impacts are designated a numerical value based on a predefined scale. While some embodiments will be described herein with particular reference to that application, it will be appreciated that the invention is not limited to such a field of use, and is applicable in broader contexts.

### BACKGROUND

Any discussion of the background art throughout the specification should in no way be considered as an admission that such art is widely known or forms part of common general knowledge in the field.

Brain injuries, particularly those sustained during participation in contact sports, are becoming an increasingly important focus of attention. For example, head impacts (and other upper body impacts) sustained during sport can have serious effects of both short term and long-term participant welfare. For example, it is valuable to better understand the nature of a suspected brain injury in terms of: (i) whether a participant should be rested from participation; (ii) an extent to which the injury should prevent a return to activity; (iii) a degree of seriousness of an injury, for instance insofar as that might affect treatment and management; and (iv) better understanding cumulative effects of successive brain injuries for a given participant.

One strategy for evaluating player impact loads as part of an injury prevention program is the use of instrumented technology. However, the implementation in the field has been limited by the reliability and validity of such technology. Using simple peak linear acceleration thresholds to differentiate impacts from normal motion is highly likely to be an insufficient method and is fraught with complex challenges. For example, setting a low magnitude acceleration threshold will increase the likelihood of false positive data, while setting a high acceleration threshold will likely result in filtering out some true impacts, while the high acceleration false positives will still remain. In addition, there are concerns that the majority of the research using sensor-recorded events lack a verification method to confirm the accuracy of the instrumented technology to identify impact loads. As a result, the absence of a verification method to confirm sensor-recorded events and to remove false positives has led to a substantial overestimation of head impact exposures.

Beyond this, there are challenges in operating instrumented mouthguard devices such that only potential impacts during use (i.e. in-mouth use) are identified. Known solutions to this problem include incorporation of additional sensor hardware into a mouthguard, including ambient light sensors, saliva sensors, and the like, which identify when a mouthguard is inserted/removed with respect to a mouth, allowing programming logic to adjust recording operation in response. These are not ideal solutions, for example in terms of added hardware complexity which needs to be embedded in a biocompatible lightweight flexible device.

### SUMMARY OF THE INVENTION

It is an object of the present invention to overcome or ameliorate at least one of the disadvantages of the prior art, or to provide a useful alternative.

One embodiment provided a method for identifying predicted head affecting impacts in data collected by an instrumented mouthguard device, the method including:
collecting time-series data from a plurality of sensors provided by the instrumented mouthguard device;
processing the time-series data thereby to define a plurality of captures based on a predefined protocol, wherein each capture includes capture event data from the plurality of sensors for a specified time period, wherein that time period is associated with a potential head affecting impact event;
processing each capture thereby to define a capture feature data set including data representative of a plurality of data features extracted from the capture event data; and
providing each capture feature data set to a classifier module, wherein the classifier module is configured to, for each capture, process the capture feature data set, and provide a classification output, wherein the classification output may include either:
   (i) output indicative of a prediction that the capture represents a head affecting impact event; or
   (ii) output indicative of a prediction that the capture represents an event other than a head affecting impact event.

One embodiment provided a method wherein processing the time-series data thereby to define a plurality of captures based on a predefined protocol includes:
processing the time-series data for one or more of the sensors thereby to identify presence of an over-threshold condition;
in the case that an over-threshold condition is identified:
   (i) commencing recording of the capture event data from a start point preceding presence of the over-threshold condition by a predefined leading period; and
   (ii) ending recording of the event data at an end point defined relative to the over-threshold condition.

One embodiment provided a method wherein processing the time-series data thereby to define a plurality of captures based on a predefined protocol includes:
processing the time-series data for one or more of the sensors thereby to identify presence of an over-threshold condition;
in the case that an over-threshold condition is identified:
   (i) commencing recording of the capture event data from a start point preceding presence of the over-threshold condition by a predefined leading period; and
   (ii) determining a point in time where an over-threshold condition is no longer detected; and
   (iii) ending recording of the event data at an end point following the point in time where an over-threshold condition is no longer detected by a predefined trailing period.

One embodiment provided a method wherein the over-threshold condition is identified by thresholding a normed signal from an accelerometer at a predefined threshold value.

One embodiment provided a method wherein processing the time-series data thereby to define a plurality of captures based on a predefined protocol includes utilising a protocol whereby a capture consists of a predefined lead in period prior to an over-threshold condition being observed, and a predefined trail time after the over-threshold condition is no longer observed.

One embodiment provided a method wherein processing the time-series data thereby to define a plurality of captures based on a predefined protocol includes a triaging process to exclude captures including vocalisation signals and/or high frequency noise.

One embodiment provided a method wherein processing the time-series data thereby to define a plurality of captures based on a predefined protocol is performed by a processor provided onboard the instrumented mouthguard device, and the capture data sets are stored in onboard memory of the instrumented mouthguard device.

One embodiment provided a method wherein processing each capture thereby to define a capture feature data set including data representative of a plurality of data features extracted from the capture event data includes any one or more of the following:
generating Convolutional Kernels;
generating Convolutional Kernels, with each signal standardised to the signal mean and standard deviation;
generating Random Convolutional Kernels;
generating Random Convolutional Kernels, with each signal standardised to the signal mean and standard deviation;

One embodiment provided a method wherein processing each capture thereby to define a capture feature data set including data representative of a plurality of data features extracted from the capture event data includes any one or more of the following:
analysing spectral characteristics;
calculating the Power Spectra Density of each signal;
splitting Power Spectra Densities into bins of defined size;
splitting Power Spectra Densities into bins of defined size, with the characteristic value of the bin extracted, then natural log transformed.

One embodiment provided a method wherein the bins of predefined size are 10 Hz bins

One embodiment provided a method wherein processing each capture thereby to define a capture feature data set including data representative of a plurality of data features extracted from the capture event data includes determining a plurality of Power Spectra Density values and a plurality of convolutional kernel features.

One embodiment provided a method wherein the step of processing each capture thereby to define a capture feature data set including data representative of a plurality of data features extracted from the capture event data is performed at a computer system remote of the instrumented mouthguard device.

One embodiment provided a method for training a classifier module to identify predicted head affecting impacts in data collected by an instrumented mouthguard device, the method including:
collecting time-series data from a plurality of sensors provided by a plurality of instrumented mouthguard devices;
processing the time-series data thereby to define a plurality of captures based on a predefined protocol, wherein each capture includes capture event data from the plurality of sensors for a specified time period, wherein that time period is associated with a potential head affecting impact event;
processing each capture thereby to define a capture feature data set including data representative of a plurality of data features extracted from the capture event data;
for each capture, labelling the capture via one or more labels, including labels representative of:
   (i) an observation based on video analysis that the capture represents a head affecting impact event; or
   (ii) an observation based on video analysis that the capture represents an event other than a head affecting impact event; and
training a classifier module based on the labelling of the captures and the capture feature data sets.

One embodiment provided a method wherein processing the time-series data thereby to define a plurality of captures based on a predefined protocol includes:
processing the time-series data for one or more of the sensors thereby to identify presence of an over-threshold condition;
in the case that an over-threshold condition is identified:
   (i) commencing recording of the capture event data from a start point preceding presence of the over-threshold condition by a predefined leading period; and
   (ii) ending recording of the event data at an end point defined relative to the over-threshold condition.

One embodiment provided a method wherein processing the time-series data thereby to define a plurality of captures based on a predefined protocol includes:
processing the time-series data for one or more of the sensors thereby to identify presence of an over-threshold condition;
in the case that an over-threshold condition is identified:
   (i) commencing recording of the capture event data from a start point preceding presence of the over-threshold condition by a predefined leading period; and
   (ii) determining a point in time where an over-threshold condition is no longer detected; and
   (iii) ending recording of the event data at an end point following the point in time where an over-threshold condition is no longer detected by a predefined trailing period.

One embodiment provided a method wherein the over-threshold condition is identified by thresholding a normed signal from an accelerometer at a predefined threshold value.

One embodiment provided a method wherein processing the time-series data thereby to define a plurality of captures based on a predefined protocol includes utilising a protocol whereby a capture consists of a predefined lead in period prior to an over-threshold condition being observed, and a predefined trail time after the over-threshold condition is no longer observed.

One embodiment provided a method processing the time-series data thereby to define a plurality of captures based on a predefined protocol includes a triaging process to exclude captures including vocalisation signals and/or high frequency noise.

One embodiment provided a method wherein processing the time-series data thereby to define a plurality of captures based on a predefined protocol is performed by a processor provided onboard the instrumented mouthguard device, and the capture data sets are stored in onboard memory of the instrumented mouthguard device.

One embodiment provided a method wherein processing each capture thereby to define a capture feature data set including data representative of a plurality of data features extracted from the capture event data includes any one or more of the following:
generating Convolutional Kernels;
generating Convolutional Kernels, with each signal standardised to the signal mean and standard deviation;
generating Random Convolutional Kernels;
generating Random Convolutional Kernels, with each signal standardised to the signal mean and standard deviation;

One embodiment provided a method wherein processing each capture thereby to define a capture feature data set including data representative of a plurality of data features extracted from the capture event data includes any one or more of the following:
analysing spectral characteristics;
calculating the Power Spectra Density of each signal;
splitting Power Spectra Densities into bins of defined size;
splitting Power Spectra Densities into bins of defined size, with the characteristic value of the bin extracted, then natural log transformed.

One embodiment provided a method wherein processing each capture thereby to define a capture feature data set including data representative of a plurality of data features extracted from the capture event data includes determining a plurality of Power Spectra Density values and a plurality of convolutional kernel features.

One embodiment provided a method wherein the step of processing each capture thereby to define a capture feature data set including data representative of a plurality of data features extracted from the capture event data is performed at a computer system remote of the instrumented mouthguard device.

One embodiment provided a method for processing data derived from an instrumented mouthguard device, the method including: (i) identifying a data set of time-series data representative of a period of time including a possible head affecting impact; (ii) processing at least a subset of that data set thereby to classify the data set as being related to a head affecting impact or otherwise; (iii) in the case that the data set is classified as head affecting impact, performing a process thereby to define a numerical value representative of magnitude of impact relative to a predefined library of impact data

Example embodiments are described below in the section entitled "claims".

Reference throughout this specification to "one embodiment", "some embodiments" or "an embodiment" means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives "first", "second", "third", etc., to describe a common object, merely indicate that different instances of like objects are being referred to, and are not intended to imply that the objects so described must be in a given sequence, either temporally, spatially, in ranking, or in any other manner.

In the claims below and the description herein, any one of the terms comprising, comprised of or which comprises is an open term that means including at least the elements/features that follow, but not excluding others. Thus, the term comprising, when used in the claims, should not be interpreted as being limitative to the means or elements or steps listed thereafter. For example, the scope of the expression a device comprising A and B should not be limited to devices consisting only of elements A and B. Any one of the terms including or which includes or that includes as used herein is also an open term that also means including at least the elements/features that follow the term, but not excluding others. Thus, including is synonymous with and means comprising.

As used herein, the term "exemplary" is used in the sense of providing examples, as opposed to indicating quality. That is, an "exemplary embodiment" is an embodiment provided as an example, as opposed to necessarily being an embodiment of exemplary quality.

### BRIEF DESCRIPTION OF THE DRAWINGS

Embodiments of the invention will now be described, by way of example only, with reference to the accompanying drawings in which:
FIG. 1A to FIG. 1D illustrates an instrumented mouthguard in varying states of assembly.
FIG. 2A and 2B illustrate an example PCB component for an instrumented mouthguard.
FIG. 3 shows the top 50 features used by a classifier model according to one embodiment.
FIG. 4 shows feature distributions of the top 5 features as defined by SHAP values, according to one embodiment.

### DETAILED DESCRIPTION

The present invention relates, in various embodiments, to automated detection of head affecting impact events in data collected via instrumented mouthguard devices. For example, in some embodiments the invention relates to training and operation of an impact classifier system, which is configured to identify head affecting impacts from time-series data collected by an instrumented mouthguard device. Some embodiments relate to a two-stage method for processing impacts, including a first stage in which a set of data is classified by such an impact classifier system, and a second stage whereby impacts classified as head impacts are designated a numerical value based on a predefined scale While some embodiments will be described herein with particular reference to that application, it will be appreciated that the invention is not limited to such a field of use, and is applicable in broader contexts.

### Context

Concussion is a common injury in contact and collision sports. There has been considerable medical interest in improving the identification and management of sport-related concussion because the accurate identification and effective medical management of concussion are important for maximising the safety and health of athletes. A number of professional sporting leagues, for example, the Australian Football League, National Football League, National Hockey League, professional rugby union, and the National Rugby League, have implemented sideline video surveillance as a strategy for improving the identification of concussion. This is an important strategy; however, concern has also been raised that it may not only be the concussive impacts that are a risk to the health of contact and collision sport athletes, but also that the accumulation of multiple impacts, that do not result in any signs or symptoms of concussion (sometimes termed 'subconcussive' impacts), that may result in current or future health issues. This notion implies that capturing data pertaining to player's body impact loads may provide important information that could be used to reduce risks to the safety and health of contact and collision sport athletes.

One strategy for evaluating player impact loads as part of an injury prevention program is the use of instrumented technology, for example instrumented mouthguard devices, thereby to identify head-affecting impacts (also referred to as Head Acceleration Events, or HAEs). However, the implementation in the field has been limited by the reliability and validity of such technology. Using simple peak linear acceleration thresholds to differentiate impacts from normal motion is highly likely to be an insufficient method and is fraught with complex challenges. For example, setting a low magnitude acceleration threshold will increase the likelihood of false positive data, while setting a high acceleration threshold will likely result in filtering out some true impacts, while the high acceleration false positives will still remain. In addition, there are concerns that the majority of the research using sensor-recorded events lack a verification method to confirm the accuracy of the instrumented technology to identify impact loads. As a result, the absence of a verification method to confirm sensor-recorded events and to remove false positives has led to a substantial overestimation of head impact exposures.

Embodiments described herein make use of machine learning technologies to enable automated classification of impacts sustained by an instrumented mouthguard device. These optionally leverage video data for the purposes of labelling training data, wherein that video data is collected in respect of players wearing instrumented mouthguard devices, and wherein the labelled training data is collected by those instrumented mouthguard devices.

### Predicting Head Affecting Impact Data in Instrumented Mouthguard Data

Embodiments include methods for identifying predicted head affecting impacts in data collected by an instrumented mouthguard device. Examples considered herein are described by reference to classification techniques which are configured to distinguish between: (i) data that is representative of a head impact event; and (ii) data that is representative something other than of a head impact, for example the mouthguard being dropped or bitten.

For the purposes of the present disclosure, the term "head affecting impact" in some embodiments is defined to include an upper body impact event which affects the head, as opposed to exclusively defining direct impacts to the head. For the purposes of this specification, the term "head impact", "head-affecting impact" "head-affecting body impact", and Head Acceleration Event (HAE)" are used interchangeably, noting that the precise definition of a relevant impact may vary between embodiments. For example, in some embodiments an upper body impact is classified as a head affecting impact for the purpose of classification. In further embodiments, the classifier may be trained based on subcategories of impact, for example including the likes of: "head", "upper body", "frontal head strike", "side head strike", "ground impact" and so on. It will be appreciated by those skilled in the art how alternate/additional labels may be added to training data based on the teachings herein.

These methods include collecting time-series data from a plurality of sensors provided by the instrumented mouthguard device. For example, the sensors may include one or more accelerometers (for example a plurality of three-axis accelerometers), and one or more gyroscopes. It should be appreciated that in further embodiments the technology may be applied to situations other than those involving the use of instrumented mouthguards.

The time-series data is recorded whilst the mouthguard is "in use", and optionally at other times when the mouthguard is in an activated state. In some embodiments a sensor is used to determine whether the mouthguard is being worn; it will be appreciated that such a sensor is not necessary in view of classifier technology disclosed herein (which identifies predicted head affecting impacts without needing to know whether the device is being worn).

The method includes processing the time-series data thereby to define a plurality of "captures" based on a predefined protocol. Each capture includes a set of capture event data derived from the plurality of sensors, for a specified time period. That time period is, as a result of the predefined protocol, predicted to be associated with a potential head affecting impact event. The step of processing the time-series data thereby to define a plurality of captures based on a predefined protocol is preferably performed by a processor provided onboard the instrumented mouthguard device, and the capture data sets are stored in onboard memory of the instrumented mouthguard device.

Processing the time-series data thereby to define a plurality of captures based on a predefined protocol includes processing the time-series data for one or more of the sensors thereby to identify presence of an over-threshold condition and, in the case that an over-threshold condition is identified:
(i) commencing recording of the capture event data from a start point preceding presence of the over-threshold condition by a predefined leading period; and
(ii) ending recording of the capture event data at an end point defined relative to the over-threshold condition. This may include determining a point in time where an over-threshold condition is no longer detected; and ending recording of the event data at an end point following the point in time where an over-threshold condition is no longer detected by a predefined trailing period.

In some embodiments, the over-threshold condition is identified by thresholding a magnitude signal from a single one of the accelerometers at a predefined threshold value (for example a value set between 4G and 12G), or in some cases a magnitude signal from more than one of the accelerometers. However, various approaches may be used, for example based on rotational velocity, gyroscope signals, and the like.

In embodiments where each a capture consists of a predefined lead in period prior to an over-threshold condition being observed, and a predefined trail time after the over-threshold condition is no longer observed, the predefined leading period is preferably between 5 ms and 30 ms (for example about 20ms), and the predefined trailing period is between 5 ms and 1000 ms (in some cases being about 200ms

In some embodiments a triaging process is implemented thereby to exclude captures including vocalisation signals and/or high frequency noise.

The method also includes processing each capture thereby to define a capture feature data set including data representative of a plurality of data features extracted from the capture event data. That is, for each capture, a plurality of features are extracted from the sensor data, and recorded in a feature data set. This may optionally be performed in a computer system remote of the instrumented mouthguard device.

The capture feature data sets include data representative of a plurality of data features extracted from the capture event data, preferably including a plurality of Power Spectra Density values and a plurality of convolutional kernel features. For example, determining the features may include any one or more of the following:
- generating Convolutional Kernels;
- generating Convolutional Kernels, with each signal standardised to the signal mean and standard deviation;
- generating Random Convolutional Kernels;
- generating Random Convolutional Kernels, with each signal standardised to the signal mean and standard deviation;
- analysing spectral characteristics;
- calculating the Power Spectra Density of each signal;
- splitting Power Spectra Densities into bins of defined size;
- splitting Power Spectra Densities into bins of defined size (for example 10Hz), with the characteristic value of the bin extracted, then natural log transformed.

The method then includes providing each capture feature data set to a classifier module, wherein the classifier module is configured to, for each capture, process the capture feature data set, and provide a classification output, wherein the classification output may include either:
(i) output indicative of a prediction that the capture represents a head affecting impact event; or
(ii) output indicative of a prediction that the capture represents an event other than a head affecting impact event.

In this manner, data is able to be continuously recorded by an instrumented mouthguard device, with a subset of the data being recorded in device memory, and that data subsequently being processed to autonomously predict head affecting impact events (as opposed to other events where the mouthguard may have experienced, for example, above threshold acceleration in a scenario which was not "head affecting"). This provides an effective tool to distinguish actual head affecting impacts from other events, for example biting the mouthguard, dropping the mouthguard, or similar.

In further embodiments, the capture data sets may include raw samples or processed data which is an output of an estimator, and/or a fast-Fourier transform of raw sample data and/or raw estimator data.

### Training of Classifier Module

The embodiment described above includes providing each capture feature data set to a classifier module, wherein the classifier module is configured to, for each capture, process the capture feature data set, and provide a classification output. That classification output may include either:
(i) output indicative of a prediction that the capture represents a head affecting impact event; or
(ii) output indicative of a prediction that the capture represents an event other than a head affecting impact event.

Embodiments also include methods for training a classifier module to identify predicted head affecting impacts in data collected by an instrumented mouthguard device. These methods preferably include:
(A) collecting time-series data from a plurality of sensors provided by a plurality of instrumented mouthguard devices;
(B) processing the time-series data thereby to define a plurality of captures based on a predefined protocol, wherein each capture includes capture event data from the plurality of sensors for a specified time period, wherein that time period is associated with a potential head affecting impact event;
(C) processing each capture thereby to define a capture feature data set including data representative of a plurality of data features extracted from the capture event data;
(D) for each capture, labelling the capture via one or more labels, including labels representative of:
   (i) an observation based on video analysis that the capture represents a head affecting impact event; or
   (ii) an observation based on video analysis that the capture represents an event other than a head affecting impact event; and
(E) training a classifier module based on the labelling of the captures and the capture feature data sets.

The time-series data is recorded whilst the mouthguard is "in use", and optionally at other times when the mouthguard is in an activated state. In some embodiments a sensor is used to determine whether the mouthguard is being worn; it will be appreciated that such a sensor is not necessary in view of classifier technology disclosed herein (which identifies predicted head affecting impacts without needing to know whether the device is being worn).

The method includes processing the time-series data thereby to define a plurality of "captures" based on a predefined protocol. Each capture includes a set of capture event data derived from the plurality of sensors, for a specified time period. That time period is, as a result of the predefined protocol, predicted to be associated with a potential head affecting impact event. The step of processing the time-series data thereby to define a plurality of captures based on a predefined protocol is preferably performed by a processor provided onboard the instrumented mouthguard device, and the capture data sets are stored in onboard memory of the instrumented mouthguard device.

Processing the time-series data thereby to define a plurality of captures based on a predefined protocol includes processing the time-series data for one or more of the sensors thereby to identify presence of an over-threshold condition. In the case that an over-threshold condition is identified, the following occurs:
(i) commencing recording (i.e. in a memory device as opposed to a temporary buffer) of the capture event data from a start point preceding presence of the over-threshold condition by a predefined leading period; and
(ii) ending recording of the event data at an end point defined relative to the over-threshold condition. This may include determining a point in time where an over-threshold condition is no longer detected; and ending recording of the event data at an end point following the point in time where an over-threshold condition is no longer detected by a predefined trailing period

In some embodiments, the over-threshold condition is identified by thresholding a magnitude signal from an accelerometer at a predefined threshold value (for example between about 4G and 12G, preferably about 8G). However, various approaches may be used, for example based on rotational velocity, gyroscope signals, and the like.

In embodiments where each a capture consists of a predefined lead in period prior to an over-threshold condition being observed, and a predefined trailing period after the over-threshold condition is no longer observed, the predefined leading period is preferable between 5 ms and 30 ms (for example about 20ms), and the predefined trailing period is between 5 ms and 1000 ms (in some cases being about 200ms).

In some embodiments a triaging process to exclude captures including vocalisation signals and/or high frequency noise.

The method also includes processing each capture thereby to define a capture feature data set including data representative of a plurality of data features extracted from the capture event data. That is, for each capture, a plurality of features are extracted from the sensor data, and recorded in a feature data set. This may be performed in a computer system remote of the instrumented mouthguard device.

The capture feature data sets include data representative of a plurality of data features extracted from the capture event data, preferably including a plurality of Power Spectra Density values and a plurality of convolutional kernel features. For example, determining the features may include any one or more of the following:
- generating Convolutional Kernels;
- generating Convolutional Kernels, with each signal standardised to the signal mean and standard deviation;
- generating Random Convolutional Kernels;
- generating Random Convolutional Kernels, with each signal standardised to the signal mean and standard deviation;
- analysing spectral characteristics;
- calculating the Power Spectra Density of each signal;
- splitting Power Spectra Densities into bins of defined size;
- splitting Power Spectra Densities into bins of defined size (for example 10Hz), with the characteristic value of the bin extracted, then natural log transformed.

By labelling the capture via labels representative of: (i) an observation based on video analysis that the capture represents a head affecting impact event (optionally with sub-labels including categories of observed head affecting impact events, such as "head clash", "ground impact", "ball impact" ,etc) ; and (ii) an observation based on video analysis that the capture represents an event other than a head affecting impact event (optionally also with sub-labels), a classifier is able to be effectively trained to enable autonomous accurate prediction of head affecting impacts.

The nature of classifier module used varies between embodiments, and preferably various algorithms are tested and/or tuned for performance comparison. In some embodiments, it is preferable to utilise an algorithm which applies a Gradient Boosting method. Those skilled in the art will recognise the "Extreme Gradient Boost" algorithm as being one example of algorithms falling within that category.

An example of how a classifier module may be trained according to some embodiments is provided below.

### Example Data Collection

In one embodiment, data for the classifier module was collected from sixty-four individuals across 119 matches in 8 clubs during the 2020 Australian Football League (AFL) season. Sixty individuals were from the men's AFL (mean age = 30.48; SD = 19.4) and four from the women's AFL (mean age = 25.50; SD = 5.91). A total of 21,348 potential impacts (captures) were generated of which 13,744 were used for training and validation purposes.

### Example Data Labelling

Ground truth data labelling on the captures used in the example embodiment was performed through analysis of game footage by two expert video reviewers using SportCode (https://www.hudl.com/en_gb/products/sportscode) and Catapult Vision (https://www.catapultsports.com/products/vision). The visual labelling process occurred independently of the mouthguard time series data. Reviewers were provided with video footage (720p, 50 frames per second) from four angles to determine if a capture (i.e. a potential impact) represented a legitimate impact - a broadcast view with a tight field of view on the ball, a side view, and footage from behind the goals. Time stamps of captures were chronologically synchronized with video footage (within +/- 1 second), and capture events were viewed and labelled according to several predefined labels. True Positive impacts (captures where the reviewer directly observed contact between the mouthguard wearer and another player, the ball, or the ground) were labelled as hits. False Positive captures (captures where no contact was observed) were given a general label (non-hit) and given a sub-label based on the activity observed-hit, biting, chewing, drinking, mouthguard insertion, mouthguard removal, mouthguard in hand, mouthguard in sock, yelling, no video footage (on sideline), and unknown (if video footage was available, but insufficient to directly observe the event).

### Instrumented Mouthguard Specifications

A HitlQ Nexus A9 instrumented mouthguard (HitlQ Pty. Ltd.) used in this study contained three triaxial accelerometers (Analog Devices ADXL372, range: ± 200G, 12-bit) and a gyroscope (Bosch BMG250, ± 2000dps range, 16-bit). These were sampled at 3200Hz and 800Hz, respectively. The circuit board and components such as a battery and antenna system were embedded in the mouthguard using a proprietary process. A three-accelerometer array located in the left, central, and right regions of the mouthguard was used to provide an estimate of angular acceleration independent of the gyroscope and allowed for a cross-check to remove spurious readings, such as those originating from actions like mouthguard deformation rather than head kinematics.

### Capture Recording

Captures were identified based on thresholding the normed signal from the left linear accelerometer at 10 g's or greater. This magnitude threshold was chosen because a magnitude significantly below 10 g's has been reported to be indicative of non-impact events (e.g., walking, sitting, etc.). A capture consisted of a lead in period 20 milliseconds prior to the 10 g threshold being reached and ended 80 milliseconds after the last trigger event. This allowed for multiple impact events to be recorded in a single capture. The capture was then stored in onboard memory in the mouthguard.

### Data Processing

Due to individual variation within linear accelerometer sampling rates, time series for each axis of the three linear accelerometer sensors were resampled to 3,200 Hz. Gyroscope data were up-sampled from 800 Hz to 3,200 Hz. All resampling was carried out using polyphase filtering as present in scipy's resample poly function.

Resampled data collections were triaged to decrease the number of vocalisation signals or those consisting of high frequency noise. The normed signal from the left linear accelerometer was low pass filtered at 300 Hz using a Butterworth 2nd order non-phase corrected filter and subject to a 10 g threshold.

Captures that passed the triage were included in the final training/validation data.

### Datasets

Data collections that passed the triage process (13,744 captures) were divided into two sets, a classifier training and validation set (Set 1) and a separate hold out set (Set 2).

Set 1 contained 13,703 captures (1,580 hits), which were balanced by downsampling the majority class (non-hit) to the minority, selecting captures to be included through pseudo-random sampling using a uniform distribution. The balanced set (3,160 captures) was divided into training (70% of the balanced data), validation (15%), and test (15%) subsets. The "rest" subset consisted of non-hit captures that were not included in the training, validation, or test subsets (10,257 non-hit captures). Set 2 consisted of captures acquired from a single match that were not included in Set 1 (57 hits, 238 non-hits).

### Classifier Design Procedure (Feature Generation)

Features were calculated on signals from all axes of the three linear accelerometers and the gyrometer (12 signals total). Signals were first aligned to cardinal axes using rotational matrices derived from a proprietary calibration process unique for each mouthguard.

Two families of features were generated to capitalise on the shape and spectral characteristics of the signals. Random Convolutional Kernels were generated, with each signal standardised to the signal mean and standard deviation. Three hundred kernels were generated, with the maximum value of the kernel and number of values greater than zero extracted per kernel. A total of 600 features were generated per signal.

Spectral characteristics were examined by calculating the Power Spectra Density of each signal, using scipy's implementation of Welch's Method. Power Spectra Densities were split into 10 Hz bins, the characteristic value of the bin extracted, then natural log transformed. The 1,908 Power Spectra Density and 720 convolutional kernel features were then standardised to the mean and standard deviation of the training set.

### Classifier Selection

Selection of a classification algorithm to use for final modelling was achieved by assessing performance of untuned algorithms on the training dataset. All available classification methods present in Scikit-learn were examined. Due to its popularity and performance, the eXtreme Gradient Boosting (XGBoost) algorithm was also included. Default settings for each algorithm were used.

The estimator with the highest true positive (TP) and true negative (TN) performance and the least difference between performance metrics in the validation set was chosen for further tuning. The least difference was included as a selection criterion to select a classification algorithm that would be unbiased towards label type.

### Classifier Training (and Performance)

RandomizedSearchCV was used to train the highest performing estimator, optimising using Matthew's Correlation Coefficient. Fifty candidate combinations of parameters were selected using 5-fold cross validation, for a total of 500 fits. The highest performing combination of hyper parameters was used for further performance validation.

Generalisability of classifier performance was assessed using TP and TN metrics and the F1 score on the validation, test, rest, and hold out data. Performance bounds were calculated using bootstrapped 95% confidence intervals, generated across 10,000 shuffles, with data selected pseudo-randomly using a uniform distribution.

### Model Interpretation

To assist with model interpretation, including insights into feature importance and the impact of features on individual observation, SHapley Additive exPlanations (SHAP)'s TreeExplainer method was used. The validation dataset was used to generate SHAP values.

### Classifier Selection Analysis

True positive (TP), true negative (TN), and the absolute difference between metrics (| TP - TN |) for all valid classifier algorithms in SKlearn and XGBoost are presented in Table 1. The mean classifier performance for TPs and TNs was 77.84% (standard deviation = 31.77%) and 89.55% (standard deviation = 11.77%) respectively, with TP values ranging from 0% (GaussianProcessClassifier, LabelPropagation, LabelSpreading, QuadraticDiscriminantAnalysis) to 98.04% (PassiveAggressiveClassifier, Perceptron) and TNs ranging from 47.53% (DummyClassifier) to 100% (GaussianProcessClassifier, LabelPropagation, LabelSpreading, QuadraticDiscriminantAnalysis). The classification algorithm that fit both selection criteria of high performance and low difference between TP and TN values was the base XGBoost classifier.

### Classifier Performance

Estimated TP, TN metrics, and the F1 score (F1) were calculated from labels estimated by the trained XGBoost against the video verified ground truth labels. Point estimate performance of the classifier was above 95% for all hit labelled impacts across all the data subsets (excluding the rest set where no TPs were present). Confidence intervals ranged from 92.51% for the test to 99.60% for the validation set. Point estimate true negative values ranged from slightly below 95% (94.54) for the hold out set to 98.65% for the validation set, while 95% Cls ranged from 91.49% (hold out set) to 100% (validation set). TP Cls suggest there was no difference between validation and test sets, while performance on the hold out set was superior (not corrected). Overlapping Cls for TNs suggests no significant difference in classifier performance across datasets.

### Model Interpretation

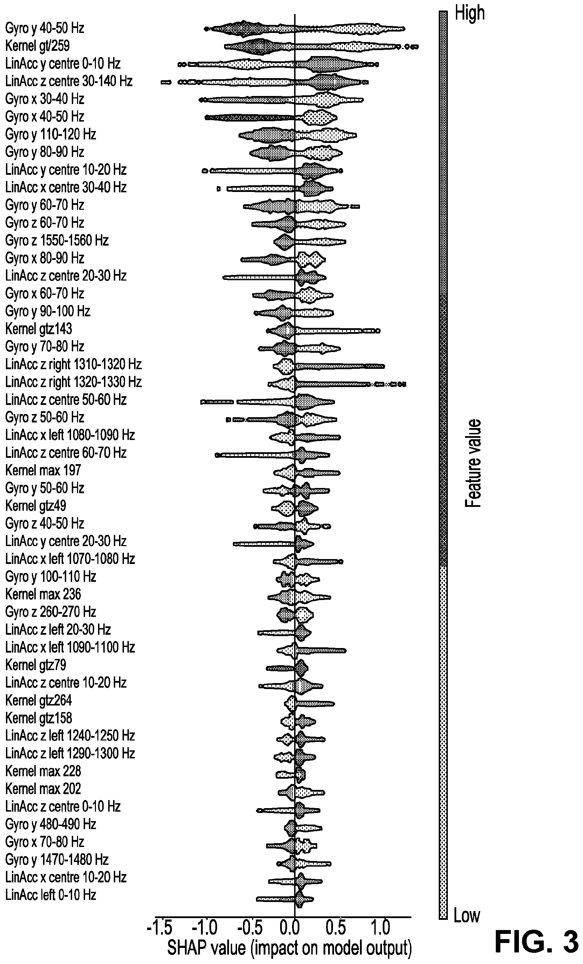

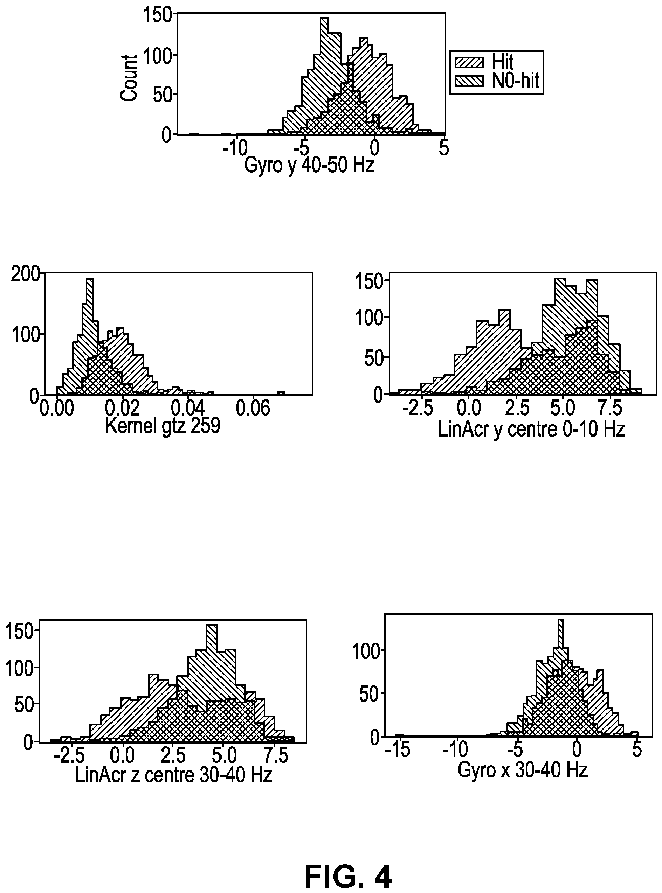

FIG. 3 shows the top 50 features used by the XGBoost model. Feature importance (y axis) goes from most important (top) in descending order of importance. Each feature has individual impacts plotted from left to right, with colour representing whether the value for that feature and observation were "high" (above the mean from memory, red) vs. "low", lower than the mean (blue), with intensity the distance. The x axis shows impact on the model. Values above 0 indicate contribution towards a positive label (hit), while values below 0 are contributions to a non-hit label. In FIG. 3, it can be seen that the top 50 features were predominantly spectral in nature with dominant frequency bands being those under 100 Hz. Gyrometer and central linear accelerometer sensors were shown to contribute the majority of information to the classifier. Feature distributions of the top 5 features as defined by SHAP values are shown in FIG. 4.

Note that in FIG. 3, Gyro = Gyrometer, LinAcc = Linear Accelerometer, x = X axis, y = Y axis, z = Z axis, Kernel = Convolutional kernel, max = Maximum value of kernel, ppv = Proportion of Positive Values in kernel. Values above 0 indicate contribution towards a positive label (hit), while values below 0 are contributions to a non-hit label. Note that in FIG. 4, SHapley Additive exPlanations (SHAP)'s TreeExplainer method was used.

### Discussion

The embodiment described above validates a body and head affecting impact detection method, based on training data derived from elite level match data of Australian Rules Football players. Data from instrumented mouthguards were combined with video verified impacts during match play to provide training and validation data for a machine learning impact classifier for Australian Rules Football. Custom generated features coupled with an XGBoost based model allowed for high performance identification of impacts and non- impacts. The reported method was trained using both on and off field captures, and thus has the ability to be used on continuously recorded data without the need to monitor only periods of on field play.

It is essential for a valid verification method to be used to confirm sensor-recorded events and to remove false positives. Video verification in combination with an algorithm-driven classifier according to embodiments described herein provides an accurate method for filtering data and optimising the integrity of the dataset. The embodiment described above showed that the classifier for the Nexus A9 mouthguard is an accurate system for identifying impacts to the body and head in elite level Australian Rules Football players.

### Two-Stage Event Classification Process

In some embodiments, a two-stage event classification process is implemented, whereby there is a first stage by which an impact event is classified as "head affecting" or "non-head affecting" as discussed above, and a second stage whereby the event data is processed thereby to define an impact value. The impact value may be, for example, a numerical value representative of relative impact magnitude, or a dosage value which is configured to be inputted to a model (for example a finite element analysis model) thereby to predict physiological outcomes of an impact.

In relation to the use of a numerical value representative of relative impact, in one embodiment a composite numerical metric is defined using a combination of accelerometer data and gyroscope data, and that metric is then benchmarked/scaled against a library of known impacts thereby to derive a simple impact magnitude value. For example, this value may be expressed as a percentage value relative to a worst-known impact in the library. Accordingly, each impact event is provided with a numerical value between 0 and 100, which is able to be used by trained medical personnel in the context of assessing a potential injurious nature or effect of an impact or series of impacts.

In a further embodiment, a technique for assigning a relative numerical value to a head affecting impact is based on: (i) determining a three-dimensional impact trajectory, including linear and rotational accelerations; (ii) using principal component analysis thereby to extract one or more key components;; and (iii) scaling the outcome relative to a distribution of values for known head affecting impact events. The outcome may optionally be a value between 1 and 10 (or another scale) which is representative of a relative magnitude/severity of head affecting impact.

### Example Instrumented Mouthguard

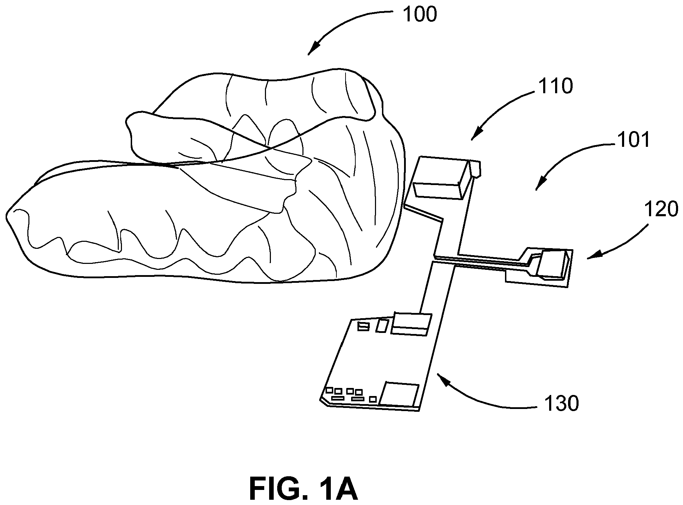

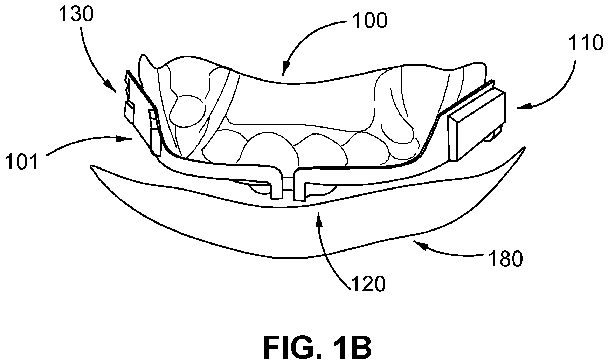

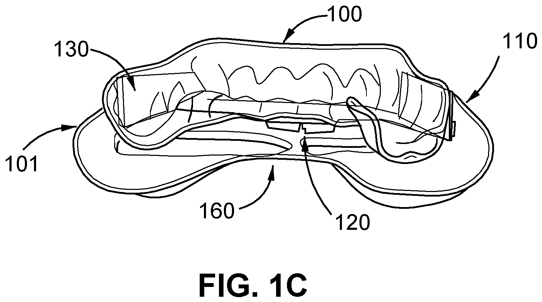

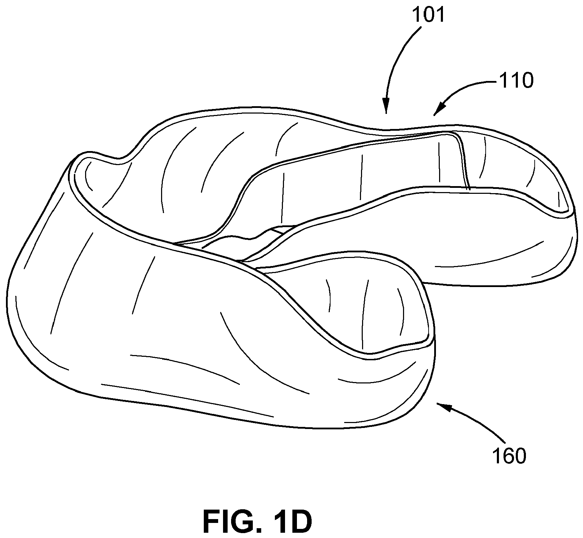

FIG. 1A to FIG. 1D illustrate an instrumented mouthguard device according to one embodiment, with features corresponding to the Nexus A9 mouthguard referred to in preceding examples. This example instrumented mouthguard is configurable to operate as a Head Impact Detection (HID) device to provide impact detection functionality. It should be appreciated that this is provided as an example only. The impact detection/classification techniques disclosed herein are able to be used with a range of different hardware configurations. For example, this may include substantially any instrumented mouthguard including minimal required sensor hardware, for example: (i) a single accelerometer and a single gyroscope; or (ii) three accelerometers. Various combinations having greater than the minimal sensor requirements are used in further embodiments.

The mouthguard comprises a mouthguard inner body 100, an instrumented component 101, and an outer mouthguard body 160. In the present embodiment the mouthguard inner body is custom formed based for a user based on a dentition scanning process, such that the mouthguard inner body provides a customised specifically to that user. The instrumented component 101 is then affixed to the inner body, and the outer body 160 sealed to the inner body 100 thereby to sandwich the instrumented component.

Additional detail regarding example instrumented mouthguard construction processes are provided in Australian provisional patent application 2020904214, entitled "multi-layered instrumented mouthguard devices, and methods for manufacturing of instrumented mouthguard devices". The disclosure of that application is hereby incorporated by cross reference.

Instrumented component 101 includes a plurality of component zones 110, 120 and 130, which are spaced apart on a flexible PCB which follows a meandering path (i.e. the distance between component zones along the PCB is greater than the direct distance between the component zones).

The meandering path allows for mounting of the flexible circuit board substrate to the mouthguard inner body, such that the component zones are located in a frontal region of the mouthguard body (component zone 120); a side region of the mouthguard inner body (component zone 110); and an opposite side region of the mouthguard inner body from the second component zone (component zone 130). The frontal region is located on an opposite side of a teeth-receiving protective channel to the side region and opposite side region. In this example the frontal region is located on an inner side of the body relative to the protective channel, and the side region and opposite side regions are located on an outer side of the body relative to the protective channel. Outer body member cover 160 is mounted to the body thereby to seal components mounted on both the outer side of the inner body relative to the protective channel thereby to cover and the inner side of the inner body relative to the protective channel.

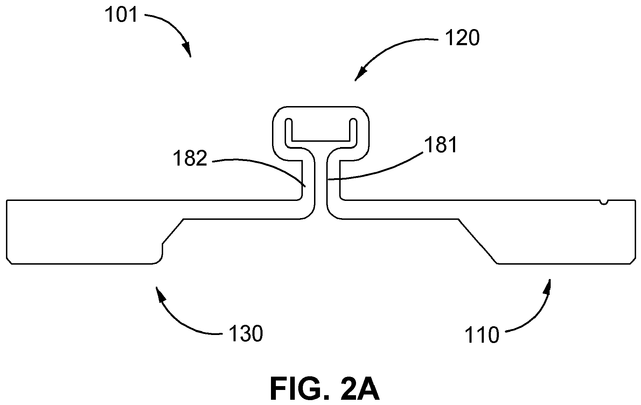

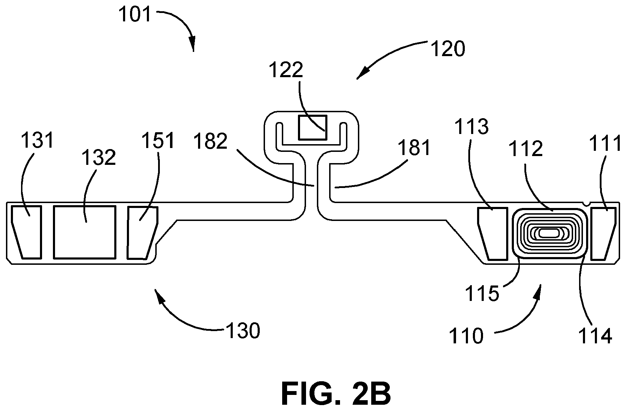

FIG. 2A and FIG. 2B illustrates an instrumented component 101 according to a further embodiment, this being configured for mounting in a mouthguard body thereby to provide an instrumented mouthguard.

As shown in FIG. 2A, component 101 is defined by a flexible circuit board substrate which is configured such that one or more conductive members electronically couples component zones (e.g. printed circuit board regions). The flexible circuit board in this manner defines a conductive member which is irregularly shaped such that it is configured to enable fitting of the component zones at desired locations on mouthguard bodies of varied shapes and sizes. More particularly, a PCB is formed to meander between component zones in a manner that allows for customisable fitting, whilst providing for added flexibility and robustness when the mouthguard is used. This presents a significant advantage over non-meandering PCBs, or the use of wires interconnecting distinct PCBs.

The PCB substrate illustrated in FIG. 2A may be of variable thickness, and/or have rigidity supports applied, thereby to adjust rigidity on a special basis thereby to protect PCB components as required for robustness.

Component 101 includes three component zones:
- A right side component zone 110. In some implementations the right side component zone is configured to support PCB components including an accelerometer(3-axis), wireless communications unit, memory and microprocessor.
- A frontal component zone 120. In some implementations, component zone 120 is split provides an accelerometer supporting zone configured to be positioned on the outer side of the front teeth (for a 3-axis accelerometer). In some embodiments the frontal zone additionally includes a low-G accelerometer and/or a gyroscope.
- A left side component zone 130. In some implementations the left side component zone provides mounting locations for an accelerometer (3-axis), battery charging unit, and a battery mounting location.
- The positioning of components described above, and shown in FIG. 2B, is an example only, and in other embodiments alternate configurations of components are distributed between the component zones.

A flexible connector member, defined by part of the PCB substrate onto which conductors connects these zones, has a first segment 181 which electronically couples right size component zone 110 and frontal component zone 120, and a second segment 182 which electronically couples front component zone 120 and left side component zone 130. As shown in FIG. 2A and 2B, these segments are meandering. In this example, as with examples above, the meandering is such that, segment 181 is greater than the length of the separation of connection points with zones 110 and 120, and segment 182 is greater than the separation of connection points with zones 120 and 130.

The flexible connector member provides a flexible substrate onto which conductive strips and a plurality of PCB components are mounted (for example PCB components in zones 110, 120 and 130). In some embodiments the flexible substrate has an increased thickness in certain regions thereby to provide increased rigidity for PCB components that are susceptible to damage as a result of PCB flexion (for example see regions 111, 112 and 113 discussed below). In some embodiments additional materials are applied to the flexible substrate thereby to increase rigidity where required.

In the embodiment of FIG. 2B, zone 110 is defined by three substantially rigid PCB regions 111, 112 and 113, interconnected by comparatively flexible regions (flex connectors) 114 and 115. This enables a better fit of zone 110 to a curved surface; in the present embodiment it is configured to mounted in a right cheek region of the mouthguard body. Zone 110 includes a range of electronic components, including:
- A 3-axis accelerometer.
- A microprocessor (for example a Qualcomm CSR1012).
- A memory module (for example a Macronix MX25L3233).
- A wireless communications module, in this embodiment being a Bluetooth module coupled to a Bluetooth antenna (not shown), for example, an antenna configured to be mounted such that it runs across a frontal region of the mouthguard forward of a wearer's teeth.
- A coupling port to a programming tab (not shown).
- A Light-Emitting Diode configured to be visible through the mouthguard body (not shown), in order to provide a device state indication to a user. For example, this is configured to be positioned behind the wearer's top lip.

It should be appreciated that the variations in rigidity within zone 110 (and across the component generally) is selected based at least in part of PCB components that are to be mounted at the various locations. For example, in one embodiment one or more of regions 111, 112 and 113 is not rigid, thereby to allow improved curvature upon application to the mouthguard body, and PCB components mounted to the non-rigid region are selected and/or mounted in such a manner to remain robust in spite to flexion in the PCB substrate.

Zone 120 includes a PCB region 122 including a 3-axis accelerometer (which is configured to be mounted to the mouthguard body in a location that in use is positioned behind front teeth). In the present embodiment PCB region 122 additionally includes a gyroscope, and a second accelerometer which is configured for lower levels of acceleration. Specifically, each component zone includes a 3-axis high-G accelerometer, and one component zone additionally includes a low-G accelerometer.

Zone 130 is configured to be mounted on a left cheek region of the mouthguard body, and includes a PCB that carries a 3-axis accelerometer 131, along with a charging coil 132 to enable wireless charging of a battery unit 151.

In other implementations the battery unit is located in zone 110 or zone 120. In further embodiments additional components including the likes of gyroscopes may also be present at one or more of the component zones (for example a gyroscope in combination with an accelerometer at each component zone.

Segment 181 of the conductive member is configured such that, upon mounting to the mouthguard body, it traverses across a bottom region of the mouthguard body at a region approximately adjacent cuspid and first bicuspid (or, alternately, first and second teeth). This allows zone 120 to be provided on an internal region (behind teeth) and zone 110 provided on an external region (in front of teeth). A sealing cover is mounted to the body thereby to seal components mounted on both the outer side of the body relative to the protective channel thereby to cover and the inner side of the body relative to the protective channel.

In a further embodiment, component 101 or a variant thereof is embedded into a post-manufacture customised (e.g. a "boil and bite") mouthguard. In such an embodiment, a standard generic form is injection moulded, and a user heats the mouthguard into a temporarily deformable state and bites firmly into it thereby to shape the resilient materials substantially to their teeth before it cools and becomes stable in the new customised shape.

### Conclusions and Interpretation

The disclosure above provides improved technology for identifying head affecting impacts via an instrumented mouthguard device. In particular, the technology allows for detected events to be classified as impact or non-impact events via an automated process, providing improved data with excludes events incorrectly predicted to be head affecting impacts.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements, if any, in the claims below are intended to include any structure, material, or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

Various aspects of the present disclosure may be embodied as a program, software, or computer instructions embodied in a computer or machine usable or readable medium, which causes the computer or machine to perform the steps of the method when executed on the computer, processor, and/or machine. A program storage device readable by a machine, tangibly embodying a program of instructions executable by the machine to perform various functionalities and methods described in the present disclosure is also provided.

A system and method of the present disclosure may be implemented and run on a general-purpose computer or special-purpose computer system. The terms "computer system" and "computer network" as may be used in the present application may include a variety of combinations of fixed and/or portable computer hardware, software, peripherals, and storage devices. The computer system may include a plurality of individual components that are networked or otherwise linked to perform collaboratively, or may include one or more stand-alone components. The hardware and software components of the computer system of the present application may include and may be included within fixed and portable devices such as desktop, laptop, and/or server. A module may be a component of a device, software, program, or system that implements some "functionality", which can be embodied as software, hardware, firmware, electronic circuitry, or etc.

Although specific embodiments of the present invention have been described, it will be understood by those of skill in the art that there are other embodiments that are equivalent to the described embodiments. Accordingly, it is to be understood that the invention is not to be limited by the specific illustrated embodiments, but only by the scope of the appended claims.

It should be appreciated that in the above description of exemplary embodiments of the invention, various features of the invention are sometimes grouped together in a single embodiment, FIG., or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

Similarly, it is to be noticed that the term coupled, when used in the claims, should not be interpreted as being limited to direct connections only. The terms "coupled" and "connected," along with their derivatives, may be used. It should be understood that these terms are not intended as synonyms for each other. Thus, the scope of the expression a device A coupled to a device B should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Thus, while there has been described what are believed to be the preferred embodiments of the invention, those skilled in the art will recognize that other and further modifications may be made thereto without departing from the spirit of the invention, and it is intended to claim all such changes and modifications as falling within the scope of the invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A method for identifying predicted head affecting impacts in data collected by an instrumented mouthguard device, the method including:
collecting time-series data from a plurality of sensors provided by the instrumented mouthguard device;
processing the time-series data thereby to define a plurality of captures based on a predefined protocol, wherein each capture includes capture event data from the plurality of sensors for a specified time period, wherein that time period is associated with a potential head affecting impact event;
processing each capture thereby to define a capture feature data set including data representative of a plurality of data features extracted from the capture event data; and
providing each capture feature data set to a classifier module, wherein the classifier module is configured to, for each capture, process the capture feature data set, and provide a classification output, wherein the classification output may include either:
(i) output indicative of a prediction that the capture represents a head affecting impact event; or
(ii) output indicative of a prediction that the capture represents an event other than a head affecting impact event.

2. A method for training a classifier module to identify predicted head affecting impacts in data collected by an instrumented mouthguard device, the method including:
collecting time-series data from a plurality of sensors provided by a plurality of instrumented mouthguard devices;
processing the time-series data thereby to define a plurality of captures based on a predefined protocol, wherein each capture includes capture event data from the plurality of sensors for a specified time period, wherein that time period is associated with a potential head affecting impact event;
processing each capture thereby to define a capture feature data set including data representative of a plurality of data features extracted from the capture event data;
for each capture, labelling the capture via one or more labels, including labels representative of:
(i) an observation based on video analysis that the capture represents a head affecting impact event; or
(ii) an observation based on video analysis that the capture represents an event other than a head affecting impact event; and
training a classifier module based on the labelling of the captures and the capture feature data sets.

3. A method according to claim 1 or claim 2 wherein processing the time-series data thereby to define a plurality of captures based on a predefined protocol includes:
processing the time-series data for one or more of the sensors thereby to identify presence of an over-threshold condition;
in the case that an over-threshold condition is identified:
(i) commencing recording of the capture event data from a start point preceding presence of the over-threshold condition by a predefined leading period; and
(ii) ending recording of the event data at an end point defined relative to the over-threshold condition.

4. A method according to any preceding claim wherein processing the time-series data thereby to define a plurality of captures based on a predefined protocol includes:
processing the time-series data for one or more of the sensors thereby to identify presence of an over-threshold condition;
in the case that an over-threshold condition is identified:
(i) commencing recording of the capture event data from a start point preceding presence of the over-threshold condition by a predefined leading period; and
(ii) determining a point in time where an over-threshold condition is no longer detected; and
(iii) ending recording of the event data at an end point following the point in time where an over-threshold condition is no longer detected by a predefined trailing period.

5. A method according to any preceding claim wherein the over-threshold condition is identified by thresholding a normed signal from an accelerometer at a predefined threshold value.

6. A method according to any preceding claim wherein processing the time-series data thereby to define a plurality of captures based on a predefined protocol includes utilising a protocol whereby a capture consists of a predefined lead in period prior to an over-threshold condition being observed, and a predefined trail time after the over-threshold condition is no longer observed.

7. A method according to any preceding claim wherein processing the time-series data thereby to define a plurality of captures based on a predefined protocol includes a triaging process to exclude captures including vocalisation signals and/or high frequency noise.

8. A method according to any preceding claim wherein processing the time-series data thereby to define a plurality of captures based on a predefined protocol is performed by a processor provided onboard the instrumented mouthguard device, and the capture data sets are stored in onboard memory of the instrumented mouthguard device.

9. A method according to any preceding claim wherein processing each capture thereby to define a capture feature data set including data representative of a plurality of data features extracted from the capture event data includes any one or more of the following:
generating Convolutional Kernels;
generating Convolutional Kernels, with each signal standardised to the signal mean and standard deviation;
generating Random Convolutional Kernels;
generating Random Convolutional Kernels, with each signal standardised to the signal mean and standard deviation;

10. A method according to any preceding claim wherein processing each capture thereby to define a capture feature data set including data representative of a plurality of data features extracted from the capture event data includes any one or more of the following:
analysing spectral characteristics;
calculating the Power Spectra Density of each signal;
splitting Power Spectra Densities into bins of defined size;
splitting Power Spectra Densities into bins of defined size, with the characteristic value of the bin extracted, then natural log transformed.

11. A method according to any preceding claim wherein processing each capture thereby to define a capture feature data set including data representative of a plurality of data features extracted from the capture event data includes determining a plurality of Power Spectra Density values and a plurality of convolutional kernel features.

12. A method according to any preceding claim wherein the step of processing each capture thereby to define a capture feature data set including data representative of a plurality of data features extracted from the capture event data is performed at a computer system remote of the instrumented mouthguard device.

13. A system for processing data derived from an instrumented mouthguard device, the method including: (i) means for identifying a data set of time-series data representative of a period of time including a possible head affecting impact; (ii) means for processing at least a subset of that data set thereby to classify the data set as being related to a head affecting impact or otherwise; (iii) means for, in the case that the data set is classified as head affecting impact, performing a process thereby to define a numerical value representative of magnitude of impact relative to a predefined library of impact data.
